(19) Europäisches Patentamt
European Patent Office
Office européen des brevets

(11) **EP 3 960 190 A1**

(12) **EUROPEAN PATENT APPLICATION**
published in accordance with Art. 153(4) EPC

(43) Date of publication:
**02.03.2022 Bulletin 2022/09**

(21) Application number: 20794798.7

(22) Date of filing: **23.04.2020**

(51) International Patent Classification (IPC):
***A61K 35/74*** *(2015.01)*     ***A61P 17/02*** *(2006.01)*

(52) Cooperative Patent Classification (CPC):
**A61K 31/7024; A61K 35/74; A61P 17/02;
C07H 5/06; C12N 1/20; C12N 1/205;
C12R 2001/365**

(86) International application number:
**PCT/CN2020/086355**

(87) International publication number:
**WO 2020/216283 (29.10.2020 Gazette 2020/44)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **24.04.2019 CN 201910333119**

(71) Applicant: **Liaoning Greatest Bio-Pharmaceutical
Co., Ltd.
Benxi, Liaoning 117004 (CN)**

(72) Inventors:
• **GAI, Bo
Benxi, Liaoning 117004 (CN)**
• **DOU, Chunyan
Benxi, Liaoning 117004 (CN)**
• **ZHANG, Yi
Benxi, Liaoning 117004 (CN)**
• **ZHANG, Guoying
Benxi, Liaoning 117004 (CN)**

(74) Representative: **Danner, Stefan
Danner BioPharm IP
Ludwigstraße 8
80539 München (DE)**

(54) **USE OF NOCARDIA RUBRA CELL WALL SKELETON IN TREATMENT OF THERMAL INJURY**

(57) Provided is the use of a *Nocardia rubra* cell wall skeleton in the preparation of a drug for treating thermal injury.

**Description**

This application claims the priority of the patent application (application number CN 201910333119.3) filed on April 25, 2019, which is incorporated herein by reference.

**TECHNICAL FIELD OF THE INVENTION**

The present disclosure relates to the fields of medicine, microbiology, and biopharmacy. Specifically, the present application relates to use of *Nocardia rubra* cell wall skeleton in the preparation of a medicament for the treatment of thermal injury.

**BACKGROUND OF THE INVENTION**

*Nocardia rubra* is a Nocardia species. The *Nocardia rubra* cell wall skeleton (hereinafter referred to as Nr-CWS or N-CWS) can be prepared by fermentation, cell disruption and protease degradation of the Nocardia rubra bacteria.

*Nocardia sp.* are polymorphous, with spherical, rod-like or filamentous shapes. The bacteria have no motility. Some strains are weakly acid-resistant, and are obligately aerobic. On ordinary agar plates, colonies can be observed after 3 days of culture, and after 7 to 10 days, the colonies bulge and form aerial hyphae with a fluffy surface. The colonies of different strains are yellow, orange or red. The molar content of G+C in DNA is 60% to 72%. Most *Nocardia sp.* are saprophytes and exist in the soil.

According to the prior art, the *Nocardia rubra* cell wall skeleton can be commercially purchased, for example, a product produced by Liaoning Greatest Bio-pharmaceutical Co., Ltd. (trade name "NAKEJIA"), or provided by Fujian M&R Pharm Co., Ltd., Fujian Cosunter Pharmaceutical Co., Ltd., Fujian Institute of Microbiology, etc. The *Nocardia rubra* cell wall skeleton has been used for the treatment of cervical erosion, cervical precancerous lesions (CN 101073583 A), anti-human papilloma virus (CN 1935262 A), skin damage (CN 101209267 A), skin lesions (eczema, neurodermatitis, non-specific dermatitis, atopic dermatitis and psoriasis) (CN 108938674 A), acne (CN 108295095 A), fungal infection, herpes simplex and herpes zoster (CN 1879661 A).

In the field of trauma surgery, the principle of anti-infective treatment is mostly used for thermal injuries, especially for the wounds caused by burns or scalds. The healing of skin thermal injury is a complex biological process, which is affected by a variety of cytokines, vascular endothelial cells, fibroblasts, keratinocytes and other various factors.

The role of *Nocardia rubra* cell wall skeleton in thermal injury has not been reported in the prior art.

**SUMMARY OF THE INVENTION**

According to some embodiments of the present application, provided is use of *Nocardia rubra* cell wall skeleton in the preparation of a medicament or a medical device for the treatment of thermal injury.

In some embodiments, the thermal injury can be caused by a factor selected from the group consisting of: burn, scald and chemical burn.

In some embodiments, the thermal injury can involve tissue(s) selected from the group consisting of: epidermis, dermis, mucosa and subcutaneous tissue.

In some embodiments, the thermal injury is a first, second (deep, superficial), or third-degree injury.

In this application, the severity of thermal injury is divided into: first degree, superficial second degree, deep second degree and third degree, according to the criteria of three degrees with four levels:

first degree: only the epidermis is injured, with local redness and swelling;
second degree: deep into the dermis, with local blisters;

- superficial second degree: only the epidermal germinal layer and dermal papillary layer are injured;
- deep second degree: the dermis is injured, with remaining skin appendages;

third degree: all layers of the skin are injured, even reaching subcutaneous tissue, deep into muscles, and bones, etc.

In particular embodiments, the *Nocardia rubra* cell wall skeleton according to the present application is especially used for the treatment of deep second-degree thermal injury.

In some embodiments, the medicament or medical device is administered through contact with the injured site.

In some embodiments, the medicament or medical device comprises a pharmaceutically acceptable carrier. Any suitable carrier known to those skilled in the art can be used to implement the technical solutions of the present application.

In some embodiments, the medicament is formulated into a dosage form selected from the group consisting of: suppository, ointment, cream, emulsion, suspension, paste, gel, lotion, tincture, oil, tablet, aerosol, spray, liniment and powder; wherein, the ointment is selected from the group consisting of: ointment, plaster and cream.

In some embodiments, provided is a method for the treatment of thermal injury, including the step: providing a subject with a therapeutically effective amount of *Nocardia rubra* cell wall skeleton.

In some particular embodiments, the medicament (or the medical device) is administered to the lesions according to the area and severity of the lesions. For example, applying a medicament comprising *Nocardia rubra* cell wall skeleton, or covering the lesion with a patch impregnated with *Nocardia rubra* cell wall skeleton, or directly applying lyophilized powder comprising *Nocardia rubra* cell wall skeleton to the lesion, applying ointment or lotion comprising *Nocardia rubra* cell wall skeleton to the lesion, etc.

In some embodiments, the medicament comprises:

- *Nocardia rubra* cell wall skeleton, and
- a pharmaceutically acceptable carrier.

In some embodiments, the pharmaceutically acceptable carrier is selected from the group consisting of: filler, stabilizer (e.g., trehalose and glycine), flavoring agent (e.g., xylitol), disintegrant (e.g., sodium carboxymethyl cellulose), binder (e.g., gelatin) and lubricant (e.g., magnesium stearate).

In some embodiments, the stabilizer is selected from one or a combination of the following: glycine, lysine, arginine, hydroxyethyl starch, hydroxymethyl starch, trehalose and dextran.

In some embodiments, the flavoring agent is selected from one or a combination of the following: sucrose, monosaccharide, sodium saccharin, aspartame, sorbitol, xylitol and mannitol.

In some embodiments, the binder is selected from one or a combination of the following: sodium carboxymethyl cellulose, hydroxypropyl methylcellulose and gelatin.

In some embodiments, the lubricant is selected from one or a combination of the following: talcum powder, magnesium stearate and micro-powder silica gel.

In some particular embodiments, as for the carrier suitable for the present disclosure, the following can be mentioned, for example: dextran, lactose, microcrystalline cellulose, trehalose, glycine, xylitol, sodium carboxymethyl cellulose, erythritol, gelatin, magnesium stearate, propellant, humectant, solvent, solubilizer, emulsifier, antioxidant, pH regulator and preservative. Specifically, non-limiting examples also include: white vaseline, carbomer, hydroxypropyl methylcellulose, methyl cellulose, sodium hydroxymethyl cellulose, chitosan, sucralfate chitosan, polyvinylpyrrolidone, polyvinyl alcohol, sodium hyaluronate, dimethyl ether, tetrafluoroethane, hydrofluoroalkane, glycerin, propylene glycol, deionized water, water for injection, distilled water, ethanol, hexadecanol, octadecanol, p-aminobenzoic acid, acetamide, isopropanol, Tween, polyoxyethyl hydrogenated castor oil, stearic acid, glyceryl monostearate, triglycerol monostearate, sucrose fatty acid ester, sucrose ester, sucrose acetate isobutyrate, sorbitan tristearate, isopropyl myristate, cholesterol, squalene, squalane, n-butanol, ethylene glycol, ethanol, propylene glycol, polyglycerol ester, sulfite, cysteine, di-tert-butyl hydroxytoluene, potassium sorbate, phosphate buffer solution, triethanolamine, sodium hydroxide, ethylenediamine, laurylamine, sodium bicarbonate, hydrochloric acid, nipagins, thimerosal, chlorocresol, trichlorobutanol, benzoic acid and sodium salt thereof.

In some embodiments, the pharmaceutically acceptable carrier is dextran.

In some embodiments, the medicament or medical device of the present application is administered 1 to 3 times a day, or once a day, or once every two days. Different doses used for each time depend on the area and degree of the patient's lesions, usually from 1 μg/unit dose/per dosing to 1000 μg/unit dose/per dosing. Specifically, for example 1, 5, 10, 15, 20, 25, 30, 35, 40, 45, 50, 55, 60, 65, 70, 75, 80, 85, 90, 95, 100, 110, 120, 130, 140, 150, 160, 170, 180, 190, 200 μg/unit dose/per dosing, and the range between any two of the preceding values.

In some embodiments, the administration cycle lasts from 2 days to 6 months, for example, 1 week, 2 weeks, 3 weeks, 4 weeks, 5 weeks, 6 weeks, 7 weeks, 8 weeks, 9 weeks, 10 weeks, 11 weeks, 12 weeks, 13 weeks, 14 weeks, 15 weeks, 16 weeks, 17 weeks, 18 weeks or longer, and the range between any two of the preceding values.

In some embodiments, the *Nocardia rubra* cell wall skeleton is a commercial *Nocardia rubra* cell wall skeleton.

In other embodiments, the *Nocardia rubra* cell wall skeleton is obtained by the following method comprising or consisting of the following steps:

(1) providing *Nocardia rubra;*
(2) disrupting the *Nocardia rubra* to obtain a disrupted product;
(3.1) optionally, removing lipids from the disrupted product;
(3.2) optionally, removing nucleic acids from the disrupted product;
(3.3) optionally, removing proteins from the disrupted product;
(3.4) obtaining a product derived from the *Nocardia rubra* cell wall;

(4) optionally, lyophilizing the product derived from the *Nocardia rubra* cell wall;
(5) optionally, performing aliquoting;

wherein steps (3.1), (3.2), and (3.3) are interchangeable in order or performed in parallel, wherein step (4) and step (5) are interchangeable in order;

wherein the average particle size of the disruption is 10 nm to 1000 nm, preferably 10 nm to 800 nm, more preferably 10 nm to 500 nm;
wherein preferably the aliquoting refers to aliquoting into containers; and wherein preferably the container is selected from the group consisting of: vial, tube, package, bag, plate, ampoule, injection device, aluminum-plastic packaging, dressing, capsule and film.

For the disruption of *Nocardia rubra,* the purpose is to remove the intracellular substances, so ultrasonication, lysozyme and other technologies can be used. The skilled person understands that any known or future method suitable for disrupting gram-positive bacteria is suitable for the technical solution of the present disclosure.

The skilled person has the ability to adjust the specific parameters and equipment for culture, disruption, separation, collection, removal of impurity, and aliquoting, according to the subsequent application (for example, external application) of the active ingredient (the cell wall and components thereof), so as to avoid introducing factors that affect the subsequent application into the preparation steps.

In some embodiments, an organic solvent is used to remove lipids from the disrupted product. In some embodiments, a nuclease is used to remove DNA and RNA from the disrupted product. In some embodiments, a hydrolase is used to degrade proteins in the disrupted product. In some embodiments, a surfactant is used to remove cell membranes from the disrupted product.

In some embodiments, the average particle size of disruption is 10 nm to 1000 nm; mention may be made of 10, 20, 30, 40, 50, 60, 70, 80, 90, 100, 110, 120, 130, 140, 150, 160, 170, 180, 190 nm $\pm$ 10 nm, and the ranges between any two of the above values. There are many methods for measuring the particle size (cf., e.g., Hu Songqing et al., Modern technology of particle size measurement, Modern Chemical Industry, 2002, 22:1).

In some particular embodiments, the average particle size of disruption is 10 nm to 800 nm. In other particular embodiments, said average particle size is 10 nm to 500 nm.

In particular embodiments, the aliquoting refers to aliquoting into vials/ampoules. A solvent (such as sterile water) is added to the vial/ampule just before use.

In some particular embodiments, the subject is an animal other than human, for example farm animal, pet, working animal, ornamental animal and production animal. In particular embodiments, the subject is a human.

In the context of the present application, the only therapeutic active ingredient in the medicament or the medical device is a product derived from *Nocardia rubra,* notably comprising the components of *Nocardia rubra* (such as proteins, nucleic acids, lipids, cell wall and components thereof, carbohydrates and metabolites); specifically, products comprising *Nocardia rubra* cell wall (more preferably *Nocardia rubra* cell wall skeleton or components thereof).

## DESCRIPTION OF THE DRAWINGS

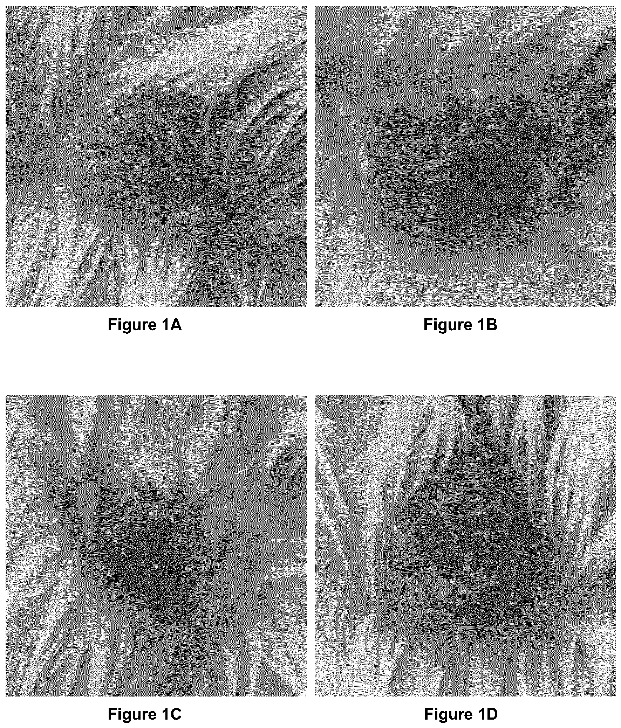

Figure 1A to Figure 1D: Healing of deep second-degree scald wounds in mice: wound on Day 21 in the broken wound treatment group (Figure 1A); wound on Day 21 in the positive group (Figure 1B); wound on Day 21 in the unbroken wound treatment group (Figure 1C); wound on Day 21 in the model group (Figure 1D).

## DETAILED DESCRIPTION OF THE EMBODIMENTS

*Nocardia rubra cell wall*

In the present disclosure, the *"Nocardia rubra* cell wall" can be interpreted as either a complete cell wall or an incomplete cell wall (for example, disrupted or partially degraded). Under the teaching of the present disclosure, the skilled person will understand that the components exhibiting the desired activity are derived from *Nocardia Rubra* cell wall (for example, the cell wall itself or components thereof). Therefore, complete cell wall, disrupted cell wall, incomplete degradation product of cell wall, cell wall components, cell wall extracts and other various forms are allowed to be used in clinical applications, which are all encompassed in the scope of the present disclosure.

*Cell wall skeleton*

A component that constitutes the main structure of the cell wall; however, it cannot be interpreted as merely representing the cross-linked network-like entity in the cell wall, and the skilled person understands that other cell wall components adsorbed by, bound to and carried by the cross-linked network-like entity are not excluded.

*Unit dose*

The medicament or the medical device of the present disclosure can be prepared in the form of a unit dose (formulation unit).

"Optionally" means that the event following this term can happen, but not necessarily happen; it depends on the situation. For example, "optionally, performing aliquoting" means that the product is allowed to be aliquoted, but is not necessary to be aliquoted; whether the product is aliquoted or not does not affect the realization of the technical effects.

"A", "an", "single" and "the", if not explicitly stated, also involve plural forms.

The present disclosure is further described below with reference to the examples. However, these examples do not limit the scope of the present disclosure. When the particular conditions are not specified, operation should be performed in accordance with the normal conditions and the conditions recommended by the raw material supplier. Reagents without giving specific sources are conventional commercial reagents.

The skilled person especially understands that although a particular commercial cell wall product is used in the following particular examples, the realization of the technical effects is not limited to the particular commercial cell wall product, and any species classified as the *Nocardia Rubra* species is applicable.

## EXAMPLES

### Example 1. Commercial *Nocardia Rubra* cell wall skeleton

The *Nocardia rubra* cell wall skeleton (trade name: NAKEJIA) was purchased from Liaoning Greatest Bio-pharmaceutical Co., Ltd., with the medicine permission No. S20030009 (2 ml/vial; lyophilized powder), which comprised 60 $\mu$g of active ingredients and 15 mg of dextran 40.

### Example 2. Preparation of *Nocardia Rubra* cell wall skeleton

1. The bacteria were cultured by well-known methods and collected. The cells were disrupted (for example, by sonication). Any appropriate well-known method in the art was also allowed to be applied in the disruption of the bacteria, for example CN 101250490 A or CN 101323865 A. The disruption state was checked under a microscope. There should be no more than 5 intact bacteria in each visual field. The disruption was considered as qualified when several (10 to 30) visual fields checked met this standard.

2. Removal of nucleic acids: the supernatant after disruption was centrifuged. DNase and RNase were added to the obtained precipitate, and nucleic acids were removed according to the operation recommended by the supplier of the enzymes.

3. Removal of proteins: commonly used protease (such as trypsin) was added to the precipitate, and proteins were removed according to the operation recommended by the supplier of the enzyme.

4. Removal of lipids: an organic reagent (for example, one or a combination of acetone, ether and ethanol) was added to the precipitate, and lipids were removed according to conventional operations in the art.

5. Removal of cell membranes: Triton X-100 was added to the precipitate, and the precipitate was collected by centrifugation according to conventional operations in the art, and rinsed with PBS.

It should be understood that among the above steps for removing impurities, those skilled in the art can adjust the order of the steps to make them compatible with each other. After removing the non-cell wall components, the precipitate was re-dissolved in water for injection, and then kept for later use. Optionally, it could be sterilized at 115°C for 20-30 minutes as the stock solution of the cell wall skeleton (mainly comprising the cell wall skeleton and components thereof).

### Example 3. Preparation of the pharmaceutical composition or medical device

1. The product obtained in Example 2 (active ingredient 60 $\mu$g to 120 $\mu$g, for example 60 $\mu$g, 70 $\mu$g, 80 $\mu$g, 90 $\mu$g, 100 $\mu$g, 110 $\mu$g, 120 $\mu$g) or the commercial product of Example 1 was coated on dressings (for example sterile gauzes) to prepare a medical device for external use.

2. Or, the product obtained in Example 2 (active ingredient 60 $\mu$g) was formulated into a lyophilized powder and applied directly on the surface of the lesion.

3. Or, a method for the preparation of lotions known in the art can also be applied, for example:

Water and ethanol are mostly used as the dispersion medium in lotion; it is prepared from active ingredients, electrolytes, isotonicity regulators, etc. in the dispersion medium. During storage of emulsion type of lotions, the oil phase and the aqueous phase may separate, but they can be re-dispersed after shaking.

**Test Example. The therapeutic effect on thermal injury**

1. Materials

1.1 Drugs and main reagents

Test drug: *Nocardia rubra* cell wall skeleton for external use (Liaoning Greatest Bio-pharmaceutical Co., Ltd., medicine permission No. S20030009, specification 60 $\mu$g/vial, batch number 201809004);

Control drug: Ching Wan Hung ointment (Tianjin Darentang Jingwanhong Pharmaceutical Co., Ltd., medicine permission No. Z20023137, specification 30 g/tube, batch number 215054);

Veet Hair Removal Cream (Reckitt Benckiser (China) Co., Ltd.);

Ether (Sinopharm Chemical Reagent Co., Ltd.).

1.2 Experimental animals

20 clean-grade Kunming mice, weighing 26 g to 35 g, were provided by Liaoning Changsheng Biotechnology Co., Ltd., with certificate number 211002300051326.

2. Experimental methods

2.1 Experimental design

20 Kunming mice were randomly divided into 4 groups, with 5 in each group:

(1) model group;
(2) unbroken wound treatment group (*Nocardia rubra* group);
(3) broken wound treatment group (*Nocardia rubra* group);
(4) positive control group (control drug).

2.2 Preparation of second-degree scald mouse model

The mice were adapted to the environment and kept for one week. After shaving the skin on the back of mice with a shaver, a depilatory cream was used to remove the hair (in an area of about 3 cm $\times$ 4 cm) and let stand for 180 s. The depilated area was washed with warm water at 30°C to 40°C and wiped dry with degreasing cotton.

After depilation, the mice were kept normally for 24 h and observed, confirming that there were no abnormal conditions such as redness, inflammation and broken skin at the depilated site. After 24 h, the mice were anesthetized with ether, placed on an operating table, the skin in the experimental area was disinfected with 75% ethanol, and the scald model was constructed.

The mice were placed on the device for scalds with a 1.2 cm hole in the middle, making the depilated skin on the back be at the place of the hole. The 1.2 cm hole with the back exposed was kept in water at a constant temperature of 75°C for 10 s, thus making a round-shaped scald wound with a diameter of about 1.2 cm.

The injured mice were kept in separate cages, the wounds were treated with saline, feed and distilled water were given, and the bedding was ensured to be dry and clean, with good ventilation. According to the criteria of three degrees with four levels, the burn/scald was identified as deep second grade.

2.3 Grouping and administration

Administration started 24 h after the scald:

(1) Model group: physiological saline was applied to the scald wound on the skin of each mouse every day;

(2) Unbroken wound treatment group: the wound was first treated with physiological saline, and then 1 vial of *Nocardia rubra* cell wall skeleton was externally applied to every animal every day (0.25 ml of physiological saline was injected into the vial to completely dissolve the lyophilized powder, which was used to completely impregnate 3 layers of 1.2 cm × 1.2 cm gauze for application on the wound, and then fixed with medical desensitization tape);

(3) Broken wound treatment group: the wound was first treated with physiological saline and made broken, and then 1 vial of *Nocardia rubra* cell wall skeleton was externally applied to every animal every day (0.25 ml of physiological saline was injected into the vial to completely dissolve the lyophilized powder, which was used to completely impregnate 3 layers of 1.2 cm × 1.2 cm gauze for application on the wound, and then fixed with medical desensitization tape);

(4) Positive control group: the wound was first treated with physiological saline, and then Ching Wan Hung ointment was externally applied to every animal once every day.

The wound healing was observed on Day 1, 3, 7, 11 and 15 of the administration.

### 3. Experimental observation and index detection

#### 3.1 Determination of wound healing time

For each group, the healing time was determined. The determination criteria include:

Healed: the scabs completely fell off at the scald site, and the surface of the repaired tissue was fresh and relatively flat;

Basically healed: the scabs intermittently fell off at the scald site, the surface of the newly grown tissue was not very flat, with a small amount of exudate in a small area but no obvious infection focus;

Infected: obvious redness and swelling appeared around the scabs, with pus or ulcer under the scab.

#### 3.2 Wound healing rate

The wound healing of mice was observed and recorded on Day 3, 7, 11 and 15 of the administration. Complete epithelialization and no exudation of the wound were regarded as complete healing of the wound. At the same time, the wound was observed for redness, swelling, infection, etc. The wound healing rate at each time point was calculated: the diameter of the wound was obtained according to the scale in the photo and by graphic processing softwares, and the wound area was calculated.

$$\text{Wound healing rate}\% = (\text{original wound area} - \text{unhealed wound area}) / \text{original wound area} \times 100\%.$$

#### 3.3 Statistical methods

SPSS 17.0 statistical software was used for analysis, and one-way ANOVA was used for comparison between groups. The data obtained was represented as $\overline{X} \pm s$. $P < 0.05$ was considered statistically significant.

### 4. Experimental results

#### 4.1 General observations

All mice survived until the wounds healed, and there were no obvious differences in eating, drinking, mental state, activity, etc., and no obvious signs of infection.

#### 4.2 Wound healing

##### 4.2.1 Wound healing time

Complete healing of the wounds and a relatively flat tissue surface of mice were considered as the standard for healing. Compared with the model group, the wound healing time of the broken wound treatment group and the positive group was shortened, and the difference was statistically significant P<0.05. Compared with the positive group, the healing time of the broken wound treatment group was slightly earlier than that of the positive group, and the difference was statistically significant P<0.05.

Table 1. Wound healing time of deep second-degree scald in mice ($\overline{X}\pm s$)

| Group | Wound healing time/d |
|---|---|
| Model group | 22.4 ± 0.3 |
| Unbroken wound treatment group | 21.6 ± 0.3 |
| Broken wound treatment group | 16.2 ± 0.2* |
| Positive group | 17.6 ± 0.3* |
| Note: compared with model group, * P<0.05. | |

4.2.2 Wound healing rate

Administration started 24 h after the model was established. The wound area before administration was set as the reference area. The wound area on Day 3, 7, 11 and 15 was recorded, and the healing rate was calculated.

Compared with the model group, the wound healing rate of the broken wound treatment group started to be significantly higher than that of the model group at Day 7 of administration, and the healing rate of the positive group and the unbroken wound treatment group also started to be higher than that of the model group at Day 11 of administration, with statistical significance P<0.05. Compared with the positive group, the wound healing rate of the broken wound treatment group started to be higher than that of the positive group at Day 7 of administration, and with statistical significance P<0.05.

Table 2. Wound healing rate of deep second-degree scald in mice ($\overline{X}\pm s$)

| Group | n | Healing rate (%) | | | |
|---|---|---|---|---|---|
| | | 3d | 7 d | 11 d | 15 d |
| Model group | 5 | 13.14±1.8 9 | 32.36±6.98 | 58.47±8.19 | 82.10±6.4 7 |
| Unbroken wound treatment group | 5 | 11.97±6.1 7 | 32.20±13.9 2 | 66.33±4.33* | 89.03±1.5 5* |
| Broken wound treatment group | 5 | 14.02±4.5 7 | 38.80:t2.93* | 76.44±4.06* | 97.83±0.1 7* |
| Positive group | 5 | 12.50±3.6 9 | 32.11±6.30 | 67.66±14.7 5* | 91.89±1.9 6* |
| Note: compared with model group, * P<0.05. | | | | | |

4.2.3 Wound healing state (Figure 1A to Figure 1D)

When the broken wound treatment group healed, the wound had no scar hyperplasia and was smooth, while other groups showed different degrees of scar hyperplasia; meanwhile, the wound site of the broken wound treatment group started to grow hair.

Without intention of being limited to particular theories, the main components of the *Nocardia rubra* cell wall skeleton are generally believed to include: muramic acid, arabinogalactan, mucopeptide, etc., which have a regulatory effect on the body's immune system and can enhance the activity of T cells, macrophages and natural killer cells, promote the production of cytokines, and have a positive effect on the early stage of wound healing and reduction of inflammation.

In the broken wound treatment group of the present application, there was basically no scar after wound healing, while other groups all had different degrees of scars. Meanwhile, the wounds of the broken wound treatment group had grown a lot of hair on Day 21, demonstrating that the composition of the present application had an extremely strong repairing effect on hair follicles and other appendages of the skin while repairing the wounds.

In summary, during the healing process of deep second-degree scalds in mice, the *Nocardia rubra* cell wall skeleton can effectively improve the healing rate, shorten the healing time, reduce the formation of scars after wound healing, while having a significant repairing effect on hair follicles and other appendages of the skin.

**Claims**

1.  Use of *Nocardia rubra* cell wall skeleton in the preparation of a medicament, wherein the medicament is for the treatment of thermal injury.

**EP 3 960 190 A1**

2. The use according to claim 1, wherein:

the thermal injury is selected from the group consisting of: burn, scald and chemical burn;
preferably, the thermal injury involves tissue(s) selected from the group consisting of: epidermis, dermis, mucosa and subcutaneous tissue;
preferably, the thermal injury is selected from the group consisting of: first degree, second degree, third degree, and more preferably deep second degree.

3. The use according to claim 1, wherein the medicament is formulated into a dosage form selected from the group consisting of: ointment, cream, emulsion, suspension, paste, gel, lotion, tincture, oil, liniment, powder, tablet, suppository, film, patch and dressing.

4. The use according to claim 1, wherein the unit dose of the medicament comprises 1 μg to 1000 μg of *Nocardia rubra* cell wall skeleton; preferably 1, 5, 10, 15, 20, 25, 30, 35, 40, 45, 50, 55, 60, 65, 70, 75, 80, 85, 90, 95, 100, 110, 120, 130, 140, 150, 160, 170, 180, 190, 200 μg.

5. The use according to claim 1, wherein the *Nocardia rubra* cell wall skeleton is a commercial *Nocardia rubra* cell wall skeleton.

6. The use according to claim 1, wherein the *Nocardia rubra* cell wall skeleton can be obtained by the following method comprising or consisting of the following steps:

(1) providing a *Nocardia rubra;*
(2) disrupting the *Nocardia rubra* to obtain a disrupted product;
(3.1) optionally, removing lipids from the disrupted product;
(3.2) optionally, removing nucleic acids from the disrupted product;
(3.3) optionally, removing proteins from the disrupted product;
(3.4) obtaining a product derived from the *Nocardia rubra* cell wall;
(4) optionally, performing aliquoting;
(5) optionally, lyophilizing the product derived from the *Nocardia rubra* cell wall;
wherein steps (3.1), (3.2), and (3.3) are interchangeable in order or performed in parallel,
wherein step (4) and step (5) are interchangeable in order;
wherein the average particle size of the disruption is 10 nm to 1000 nm, preferably 10 nm to 800 nm, more preferably 10 nm to 500 nm;
wherein preferably the aliquoting refers to aliquoting into containers; and
wherein preferably the container is selected from the group consisting of: vial, tube, package, bag, plate, ampoule, injection device, aluminum-plastic packaging, dressing, capsule and film.

7. A method for the treatment of thermal injury, including the following steps:

making a subject in contact with a therapeutically effective amount of *Nocardia rubra* cell wall skeleton;
the *Nocardia rubra* cell wall skeleton is formulated into a form selected from the group consisting of: ointment, cream, emulsion, suspension, paste, gel, lotion, tincture, oil, liniment, powder, tablet, suppository, film, patch and dressing;
the contact is performed by administering twice a day, or once a day, or once every two days, or once every three days, or once a week;
the contact lasts for 2 days, 3 days, 4 days, 5 days, 6 days, 1 week, 2 weeks, 3 weeks, 4 weeks, 5 weeks, 6 weeks, 7 weeks, 8 weeks, 9 weeks, 10 weeks, 11 weeks, 12 weeks, 13 weeks, 14 weeks, 15 weeks, 16 weeks, 17 weeks, 18 weeks or longer;
the thermal injury is selected from the group consisting of: burn, scald and chemical burn;
preferably, the thermal injury involves tissue(s) selected from the group consisting of: epidermis, dermis, mucosa and subcutaneous tissue;
preferably, the thermal injury is selected from the group consisting of: first degree, second degree, third degree; and most preferably, deep second degree.

8. The method according to claim 7, wherein the *Nocardia rubra* cell wall skeleton is a commercial *Nocardia rubra* cell wall skeleton.

9. The method according to claim 7, wherein the *Nocardia rubra* cell wall skeleton is obtained by the following method comprising or consisting of the following steps:

(1) providing *Nocardia rubra;*
(2) disrupting the *Nocardia rubra* to obtain a disrupted product;
(3.1) optionally, removing lipids from the disrupted product;
(3.2) optionally, removing nucleic acids from the disrupted product;
(3.3) optionally, removing proteins from the disrupted product;
(3.4) obtaining a product derived from the *Nocardia rubra* cell wall;
(4) optionally, performing aliquoting;
(5) optionally, lyophilizing the product derived from the *Nocardia rubra* cell wall;
wherein steps (3.1), (3.2), and (3.3) are interchangeable in order or performed in parallel,
wherein step (4) and step (5) are interchangeable in order;
wherein the average particle size of the disruption is 10 nm to 1000 nm, preferably 10 nm to 800 nm, more preferably 10 nm to 500 nm;
wherein preferably the aliquoting refers to aliquoting into containers; and
wherein preferably the container is selected from the group consisting of: vial, tube, package, bag, plate, ampoule, injection device, aluminum-plastic packaging, dressing, capsule and film.

Figure 1A

Figure 1B

Figure 1C

Figure 1D

## INTERNATIONAL SEARCH REPORT

| International application No. |
| --- |
| **PCT/CN2020/086355** |

| A. | CLASSIFICATION OF SUBJECT MATTER |
| --- | --- |

A61K 35/74(2015.01)i; A61P 17/02(2006.01)i

According to International Patent Classification (IPC) or to both national classification and IPC

| B. | FIELDS SEARCHED |
| --- | --- |

Minimum documentation searched (classification system followed by classification symbols)

A61K; A61P

Documentation searched other than minimum documentation to the extent that such documents are included in the fields searched

Electronic data base consulted during the international search (name of data base and, where practicable, search terms used)

数据库: CNABS, VEN(DWPI+SIPOABS), CNTXT, EPTXT, USTXT, WOTXT, CNKI, PubMed, Elsevier Science, ISI-WEB OF SCIENCE; 检索词: 红色诺卡氏菌, 红色诺卡氏菌细胞壁骨架, 纳可佳, 热损伤, 烧伤, 烫伤, 灼伤, Nocardia rubra, Nr-CWS, N-CWS, cell wall skeleton, burn, scald

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| X | CN 101209267 A (SHENYANG SHENGBAO BIOLOGICAL PHARMACEUTICAL CO., LTD.) 02 July 2008 (2008-07-02) see claims 2, 6, 9-14, description, page 4, line 15 to page 6, line 7, page 7, line 2 (from the bottom) to page 9, line 19, table 5 | 1-6 |
| A | Yi Wang et al. "Nocardia rubra cell wall skeleton accelerates cutaneous wound healing by enhancing macrophage activation and angiogenesis" *J Int Med Res.*, Vol. 46, No. 6, 18 April 2018 (2018-04-18), ISSN: 1473-2300, see entire document | 1-6 |

☐ Further documents are listed in the continuation of Box C.    ☑ See patent family annex.

| * | Special categories of cited documents: | | "T" | later document published after the international filing date or priority date and not in conflict with the application but cited to understand the principle or theory underlying the invention |
| --- | --- | --- | --- | --- |
| "A" | document defining the general state of the art which is not considered to be of particular relevance | | | |
| "E" | earlier application or patent but published on or after the international filing date | | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | | |
| "P" | document published prior to the international filing date but later than the priority date claimed | | "&" | document member of the same patent family |

| Date of the actual completion of the international search | Date of mailing of the international search report |
| --- | --- |
| **27 May 2020** | **10 June 2020** |

| Name and mailing address of the ISA/CN | Authorized officer |
| --- | --- |
| **China National Intellectual Property Administration (ISA/CN)** **No. 6, Xitucheng Road, Jimenqiao Haidian District, Beijing 100088** **China** | |
| Facsimile No. **(86-10)62019451** | Telephone No. |

Form PCT/ISA/210 (second sheet) (January 2015)

**INTERNATIONAL SEARCH REPORT**

| International application No. |
|---|
| **PCT/CN2020/086355** |

| Box No. II | Observations where certain claims were found unsearchable (Continuation of item 2 of first sheet) |
|---|---|

This international search report has not been established in respect of certain claims under Article 17(2)(a) for the following reasons:

1. ☑ Claims Nos.: **7-9**
   because they relate to subject matter not required to be searched by this Authority, namely:

   [1]   Claims 7-9 relate to a method for treatment of the human or animal body.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (January 2015)

**INTERNATIONAL SEARCH REPORT**
Information on patent family members

International application No.

**PCT/CN2020/086355**

| Patent document cited in search report | | | Publication date (day/month/year) | Patent family member(s) | | | Publication date (day/month/year) |
|---|---|---|---|---|---|---|---|
| CN | 101209267 | A | 02 July 2008 | CN | 101209267 | B | 03 October 2012 |

Form PCT/ISA/210 (patent family annex) (January 2015)

14

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 201910333119
- CN 101073583 A
- CN 1935262 A
- CN 101209267 A
- CN 108938674 A
- CN 108295095 A
- CN 1879661 A
- CN 101250490 A
- CN 101323865 A

**Non-patent literature cited in the description**

- **HU SONGQING et al.** Modern technology of particle size measurement. *Modern Chemical Industry,* 2002, vol. 22, 1